# EUROPEAN PATENT APPLICATION

(11) **EP 1 240 819 A1**
(43) Date of publication of application: **18.09.2002**
(21) Application number: 99974207.5
(22) Date of filing: 19.11.1999
(51) Int. Cl.: A01H 5/00, C12N 15/82

(54) **METHOD FOR LOWERING POLLEN FERTILITY BY USING TAPETAL LAYER-SPECIFIC ZINC FINGER TRANSCRIPTIONAL FACTOR GENE**

(71) Applicant: National Institute of Agrobiological Sciences, Tsukuba-shi, Ibaraki 305-8602 (JP); Bio-oriented Technology Research Advancement Institution, Omiya-shi, Saitama 331-8537 (JP)
(72) Inventor: KAPOOR, Sanjay, Tsukuba-shi Ibaraki 305-0035 (JP); KOBAYASHI, Akira, D-204 Nogyoshikenjoshukusha, Menuro-cho, Kasai-gun, Hokkaido 082-0052 (JP); TAKATSUJI, Hiroshi, Nat. Inst. of Agrobio. Scienc., Tsukuba-shi, Ibaraki 305-8602 (JP)
(74) Representative: Harding, Charles Thomas
(86) International application number: JP9906468
(87) International publication number: WO01037644

(57) **Abstract**

A method is provided for producing a male sterile plant by utilizing a plant expression cassette including a nucleic acid which is DNA encoding a zinc finger transcription factor (ZPT3-2) derived from Petunia and a promoter operatively linked to the nucleic acid. Further, a method is provided for producing a male sterile plant by utilizing a plant expression cassette including a promoter derived from the ZPT3-2 gene and a heterologous gene operatively linked to the promoter.

## Description

### TECHNICAL FIELD

The present invention relates to a gene which is expressed specifically in the anther of stamens and use of the same. More particularly, the present invention relates to the gene for a zinc finger transcription factor (ZPT3-2) derived from Petunia, which is expressed specifically in the tapetum of an anther, and use of the same.

### BACKGROUND ART

Pollen fertility causes problems in various aspects of agriculture and horticulture. For example, in the case of mating for cross breeding, self-pollination has to be avoided by castration (removal of stamens) which requires enormous effort. In the seed and seedling industry, there is a demand for a trait of lack of pollen fertility from the standpoint of commercially protecting excellent breeds obtained by cross breeding. To meet such a demand, a technique for controlling pollen fertility (pollination control) has been strongly required. Conventionally, for particular crops, lines of cytoplasmic male sterility have been used for cross breeding, and some success has been achieved. However, the cytoplasmic sterility trait is often accompanied by undesired side effects, such as a reduction in disease resistance and the like. There are further problems, such as that the trait is unstable, that it is difficult to mass-produce the seeds, and the like. A method for reducing the fertility by treating with a chemical agent(s) has been studied, but safety evaluation and elucidation of the mechanism of this method have not been fully done and thus such a method is not yet in actual use. Therefore, there is a demand for an excellent male sterilization technique using genetic engineering.

Tapetum is a tissue which is located at the innermost side of the wall of an anther and surrounds sporogenous cells (pollen cells). Tapetum plays an essential role in the development of pollen. Tapetum has a variety of functions, i.e., it not only supports pollen cells but also supplies nutrients necessary for the development of pollen, degrades a callose layer including tetrads, supplies compounds constituting the exine layer of the surface of a pollen cell, and the like. The development of an anther is divided into the following stages: the tetrad stage immediately after the meiosis of sporogenous cells; the release stage during which microspores are released from the tetrad; the uninucleate stage characterized by the enlargement and vacuolation of pollen cells, the mitotic stage giving rise to the differentiation into vegetative and generative cells by mitosis; and the subsequent binucleate stage. After these stages, the anther finally dehisces and matured pollen grains are released. It is known that if only a part of the functions of a tapetum is impaired in the above-described developmental processes, the development of most normal pollens is inhibited, so that pollen fertility is eliminated.

As described above, great expectations are placed on male sterilization techniques using genetic engineering. Particularly, if a gene which is expressed specifically in a small region which is not exposed outside, such as a tapetum, can be utilized, it is considered to be highly likely that male sterilization can be achieved without conferring undesired traits to plants. Several examples of promoters specific to a tapetum and gene constructs for male sterilization comprising the promoter have been reported (Shivanna and Sawhney Ed., Pollen biotechnology for crop production and improvement (Cambridge University Press), pp. 237-257, 1997). However, there is still a demand for a novel gene useful for control of pollen fertility, which has high tissue and temporal specificities of expression.

Recently, the inventors of the present application specified the cDNA sequences of novel transcription factors derived from Petunia, i.e., seven zinc finger (ZF) transcription factors including PEThy ZPT3-2 (hereinafter abbreviated as ZPT3-2). And the inventors reported that Northern blot analysis indicates that each transcription factor transiently expresses in an anther-specific manner in a different stage of the development of the anther (Kobayashi et al., Plant J., 13:571, 1998). However, the physiological function and action of these transcription factors in plants, and the precise expression sites and the expression controlling mechanism of the genes encoding the transcription factors have been not clarified.

### DISCLOSURE OF THE INVENTION

The objective of the present invention is to provide a genetic engineering technique using an anther tissue-specific gene which is useful for modification of a plant trait, representatively male sterility.

The present inventors reintroduced a gene encoding one of anther-specific transcription factors (ZPT3-2), which had been previously isolated from Petunia, into Petunia. As a result, it was found that the normal development of pollen was inhibited by inhibiting the expression of an endogenous gene by the introduced gene, so that pollen fertility was significantly reduced. Further, the inventors isolated upstream regions of the ZPT3-2 genomic gene, and studied the tissue specificity of the promoter activity. As a result, it was found that the promoter activity is expressed in a tapetum from the tetrad stage to the uninucleate stage in a tissue and temporal-specific manner. The present invention was completed based on these findings.

According to a first aspect of the present invention, a method for producing a male sterile plant comprises the steps of providing a plant expression cassette including: a nucleic acid being any of (i) DNA having a sequence from position 1 to position 1886 of a base sequence indicated by SEQ ID NO: 1, (ii) DNA hybridizing the DNA having the base sequence (i) under stringent conditions and encoding a transcription factor controlling the development of pollen, and (iii) a DNA fragment of (i) or (ii); and a promoter operatively linked to the nucleic acid, providing plant cells having an endogenous transcription factor controlling the development of pollen, wherein a gene encoding the endogenous transcription factor hybridizes the nucleic acid under stringent conditions, introducing the expression cassette into the plant cells, regenerating the plant cells, into which the expression cassette has been introduced, to plants; and screening the regenerated plants for one in which the nucleic acid is expressed so that expression of the endogenous transcription factor is suppressed. The method may be utilized as a method for conferring male sterility to a plant.

In one embodiment, the nucleic acid is linked in a forward direction with respect to the promoter, and may be transcribed in a sense direction in cells of the plant.

In one embodiment, the nucleic acid is linked in a reverse direction with respect to the promoter, and may be transcribed in a antisense direction in cells of the plant.

In one embodiement, the plant is dicotyledon. The dicotyledon is preferably of the family Solanaceae, and more preferably of the genus Petunia.

In one embodiment, the expression cassette is incorporated into a plant expression vector.

According to the first aspect of the present invention, a male sterile plant produced by a method according to any of the above-described methods is provided.

According to a second aspect of the present invention, a method for producing a male sterile plant comprises the steps of providing a plant expression cassette including: a promoter including any of (a) DNA having a sequence from position 1 to position 1991 of a base sequence indicated by SEQ ID NO: 3 and (b) DNA having a part of the sequence of (a) and exhibiting promoter activity specific to the tapetum of an anther; and a heterologous gene operatively linked to the promoter, introducing the expression cassette into plant cells, and regenerating the plant cells, into which the expression cassette has been introduced, to plants. The method may be utilized as a method for conferring male sterility to a plant.

In one embodiement, the plant is dicotyledon. The dicotyledon is preferably of the family Solanaceae, and more preferably of the genus Petunia.

In one embodiment, the expression cassette is incorporated into a plant expression vector.

According to the second aspect of the present invention, a male sterile plant produced by a method according to any of the above-described methods is also provided.

According to a third aspect of the present invention, a promoter comprises DNA of the following (I) or (II): (I) DNA having a sequence from position 1 to position 1991 of a base sequence indicated by SEQ ID NO: 3; and (II) DNA having a part of the sequence of (I) and exhibiting promoter activity specific to the tapetum of an anther.

According to a fourth aspect of the present invention, a plant expression cassette useful for conferring male sterility to a plant comprises the promoter and a heterologous gene operatively linked to the promoter.

### BRIEF DESCRIPTION OF THE DRAWINGS

Figure **1** is a diagram showing a cDNA sequence of a gene encoding ZPT3-2 (herein also simply referred to as "ZPT3-2 gene") and the corresponding amino acid sequence. Three zinc finger motifs and a DLNL sequence (amino acids from position 411 to position 425) are underlined.
Figure **2** is a schematic diagram showing the structure of a plant expression vector used for expression of the cDNA sequence of ZPT3-2 (pBIN-35S-ZPT3-2).
Figure **3** is a diagram showing an upstream sequence of the coding region of the ZPT3-2 gene. The transcription initiation site is indicated by a thick arrow (position 1721). The translation initiating codon (ATG) is indicated by a thick underline.
Figure **4** is a schematic diagram showing the structure of a plant expression vector for analyzing a promoter for the ZPT3-2 genes (pBIN-ZPT3-2-GUS).

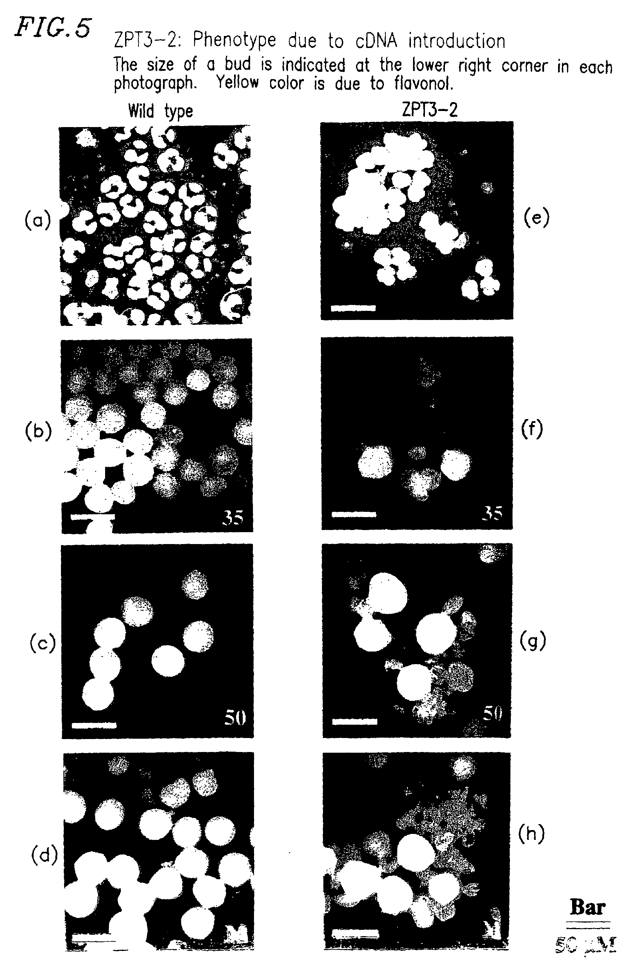

Figure **5** shows photographs indicating the forms of an organism, i.e., the pollens of a wild-type Petunia and the pollens of Petunia into which pBIN-35S-ZPT3-2 was introduced (magnification is 240 times). All of the pollens were stained with flavonol. Figures **5(a)** through **5(d)** show pollens at the different growth stages of the bud of awild-type Petunia. Figures **5(e)** through **5(h)** show pollens at the different growth stages of the bud of a transformed Petunia. Figures **5(a)** and **5(e)** show the pollens of a bud having a size of 5 mm. Figures **5(b)** and **5(f)** show the pollens of a bud having a size of 35 mm. Figures **5(c)** and **5(g)** show the pollens of a bud having a size of 50 mm. Figures **5(d)** and **5(h)** show the pollens at a matured stage.
Figure **6** shows photographs indicating the form of the organisms, i.e., GUS-stained floral organs of Petunia into which pBIN-ZPT3-2-GUS was introduced. Each photograph was taken of a flower (bud) whose anther is in the tetrad stage. Figure **6(a)** shows the appearance of the bud at a magnification of 2.5. Figure **6(b)** shows a cross-sectional view of an anther at a low magnification (60 times). Figure **6(c)** shows a cross-sectional view of the tapetum of an anther and the surrounding vicinity of the tapetum at a high magnification (280 times).

### BEST MODE FOR CARRYING OUT THE INVENTION

Hereinafter, the present invention will be described in detail.

### (Transcription factor derived from ZPT3-2 gene)

A nucleic acid, which is useful in a method for producing male sterile plants according to a first aspect of the present invention, is any one of the following DNAs: (i) DNA having a sequence from position 1 to position 1886 of a base sequence indicated by SEQ ID NO: 1; (ii) DNA which hybridizes to the DNA having the base sequence (i) under stringent conditions, and encodes a transcription factor which controls the development of pollen; or (iii) DNA which is a fragment of any of the above-described DNAs (i.e., (i) or (ii)). The above-described nucleic acid of the present invention is preferably DNA of (i), i.e., DNA encoding ZPT3-2, or a fragment thereof, and more preferably DNA of (i).

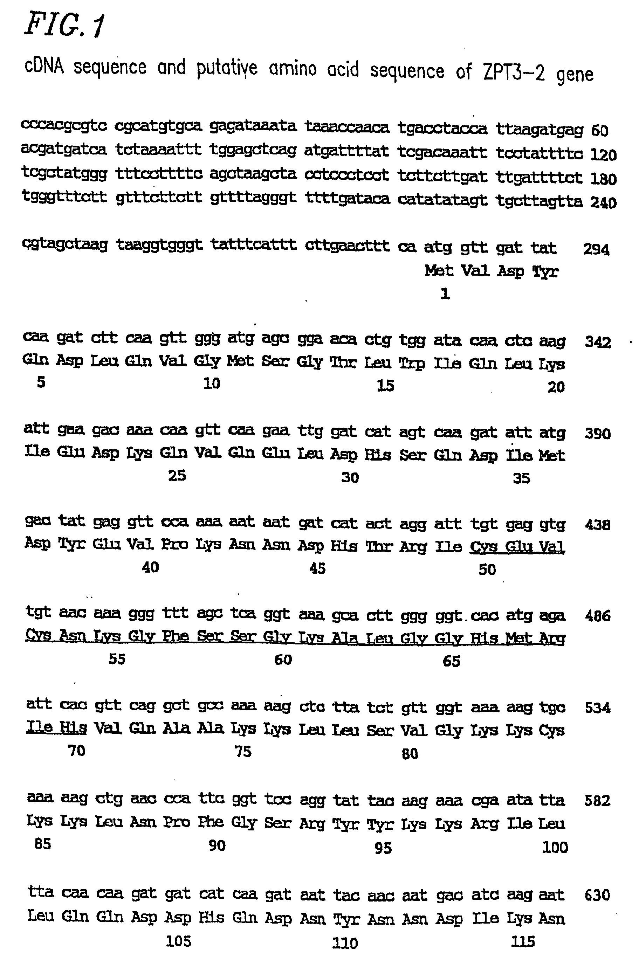

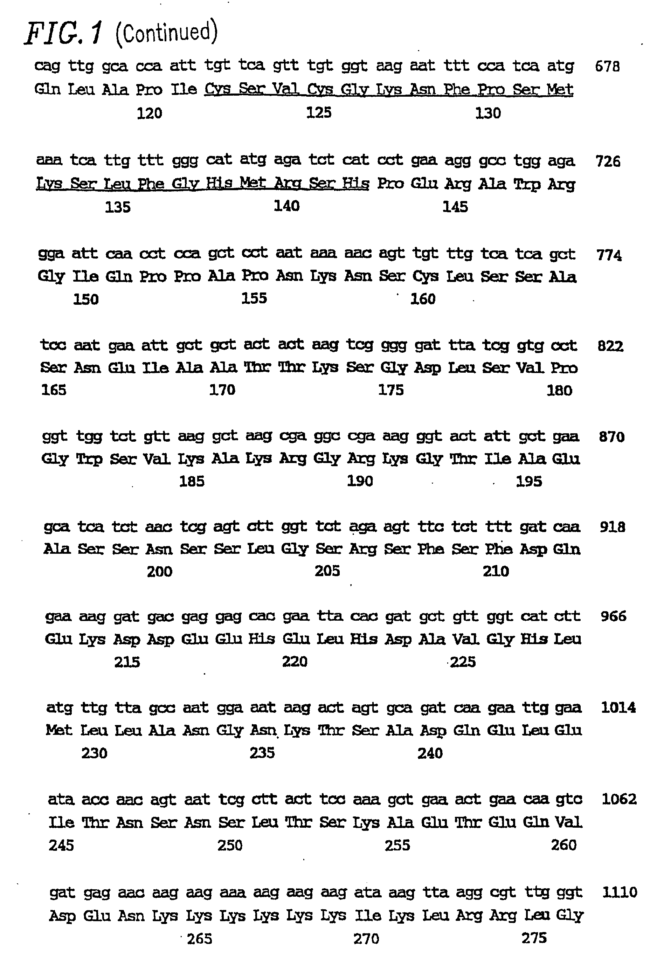

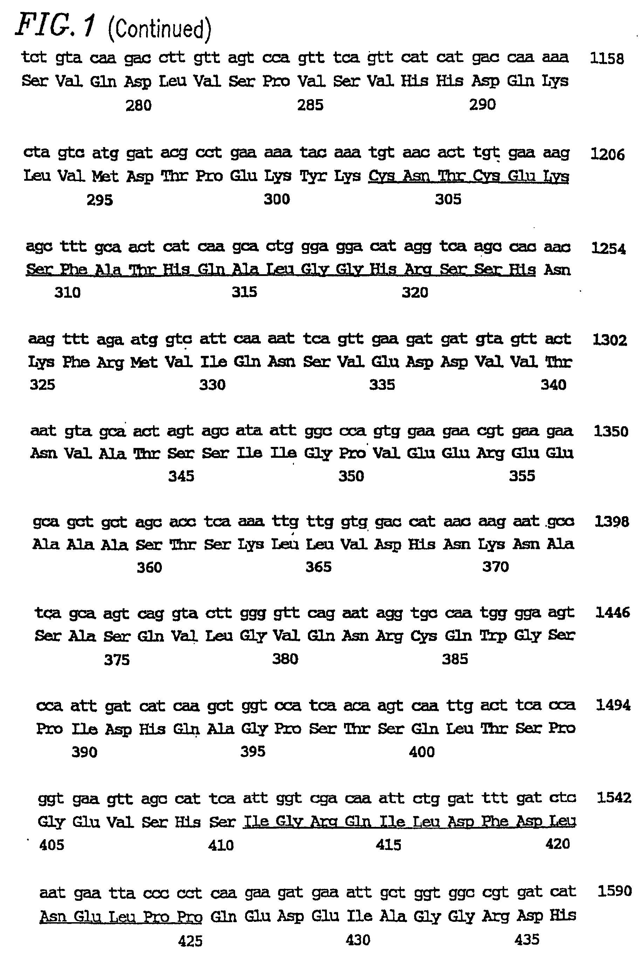

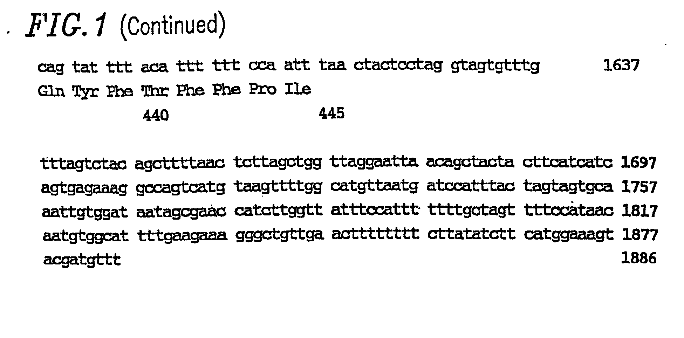

In the present specification, "transcription factor" refers to a protein for controlling the synthesis of mRNA by binding to DNA in the regulatory region of a gene. It is known that a certain type of transcription factor has a highly conserved amino acid sequence called a zinc finger motif in the DNA binding domain. ZPT3-2 is a zinc finger protein of the Cys2/His2 type (EPF family), which is a transcription factor which includes three zinc finger motifs in the full-length amino acid sequence consisting of 444 amino acids, and further, a hydrophobic region called a DLNL sequence (Kobayashi et al. above). A cDNA sequence encoding ZPT3-2 (SEQ ID NO: 1) is indicated in Figure **1** along with the corresponding putative amino acid sequence (SEQ ID NO: 2)

In the present specification, "fragment" of a nucleic acid or DNA refers to a fragment which can inhibit the expression of an endogenous transcription factor in a plant when the fragment is introduced into the plant and expressed in an appropriate manner. This fragment is selected from regions of DNAs of the above-described (i) or (ii) other than the regions encoding the zinc finger motifs in the DNAs. The fragment has a length of at least about 40 bases or more, preferably about 50 bases or more, more preferably about 70 bases or more, and even more preferably about 100 bases or more.

In the present specification, "stringent conditions" for hybridization are intended as conditions sufficient for the formation of a double-strand oligonucleotide of a particular base sequence (e.g., DNA encoding ZPT3-2 derived from Petunia) and another base sequence having a high level of homology with the particular base sequence (e.g., DNA encoding a homolog of ZPT3-2 which is present in a plant other than Petunia). A representative example of the stringent conditions applied to the present invention are the following: hybridization is conducted in a solution containing 1M NaCl, 1%SDS, 10% dextran sulfate, ³²P-labeled probe DNA (1×10⁷ cpm) and 50 µg/ml salmon sperm DNA at 60°C for 16 hours, followed by washing twice with 2×SSC/1%SDS at 60°C for 30 minutes.

In the present invention, a degenerate primer pair corresponding to a conserved region of an amino acid sequence encoded by the gene of a known transcription factor may be used in order to isolate DNA encoding ZPT3-2, and DNA encoding a transcription factor which hybridizes that DNA under stringent conditions to inhibit the development of pollen. PCR is conducted using this primer pair with cDNA or genomic DNA of a plant as a template, thereafter, the resultant amplified DNA fragment is used as a probe so that the cDNA or genomic library of the same plant can be screened. As an example of such a primer pair, a combination of 5'-CARGCNYTNGGNGGNCAY-3' (SEQ ID NO: 4), and 3'-RTGNCCNCCNARNGCYTG-5' (SEQ ID NO: 5) is illustrated (where N indicates inosine, R indicates G or A, and Y indicates C or T). The above-described primer sequences each correspond to an amino acid sequence QALGGH included in the zinc finger motif of ZPT3-2.

Therefore, the stringent hybridization conditions which are applied to the present invention may also be used for PCR. In a representative example, the above-described degenerate primer (SEQ ID NOs: 4 and 5) may be used. In this case, the PCR reaction conditions may be the following: denaturation at 94°C for 5 minutes; followed by 30 cycles of 30 seconds at 94°C, 30 seconds at 50°C and 1 minute at 72°C; and finally, incubation at 72°C for 7 minutes.

PCR may be conducted based on the manufacturer's instruction for a commercially available kit and device, or a method well known to those skilled in the art. A method for preparing a gene library, a method for cloning a gene, and the like are also well known to those skilled in the art. For example, see Sambrook et al., Molecular Cloning: A Laboratory Manual, 2nd Ed. (Cold Spring Harbor Laboratory, 1989). The base sequence of a resultant gene may be determined with a nucleotide sequencing analysis method known in the art, or by a commercially available automatic sequencer.

In the present specification, "controlling the development of pollen" by a transcription factor representatively means that when the expression of this transcription factor is inhibited, a significant change in the form or functions of pollen is observed. Representatively, by inhibiting the expression of a gene encoding the transcription factor of the present invention, preferably about 60% or more, more preferably about 75% or more, and even more preferably about 90% or more of pollen cells are killed before being matured. When the amount of mRNA measured by a Northern blot method is about one tenth or less as compared to a wild-type control plant, the expression of a transcription factor is judged to be inhibited.

Whether or not the transcription factors encoded by genes isolated and identified by screening as above (i.e., ZPT3-2 and the homologs of ZPT3-2) control the development of pollen, can be confirmed by producing a transformed plant and observing the characteristics of the pollen of the plant in accordance with the disclosure of the present specification.

According to the present invention, DNA encoding a transcription factor which controls the development of pollen, or a fragment thereof, can be utilized to inhibit the expression of an endogenous gene having the same orhomologous base sequence as that of the DNA in plant cells. Such a target endogenous gene is also a transcription factor which controls the development of pollen. According to the method of the present invention, plants are conferred male sterility by selectively inhibiting only the expression of an endogenous transcription factor, preferably without substantially inhibiting the expression of genes other than the endogenous transcription factor which controls the development of pollen.

In other words, plant cells to which the expression inhibiting technique of the present invention is applied are plant cells having an endogenous transcription factor which controls the development of pollen. The gene encoding this endogenous transcription factor is defined as a gene which hybridizes with DNA encoding the above-described ZPT3-2 or a homolog of ZPT3-2 under stringent conditions. The definition of the "stringent conditions" is the same as that described in relation to specification of the homolog of ZPT3-2. Plants capable of being conferred male sterility with the above-described method are preferably plants which are phylogenetically, closely related to Petunia from which the ZPT3-2 gene is isolated, or plants from which genes encoding the above-described ZPT3-2 homologs are isolated, but the present invention is not intended to be limited to this. "Plants which are phylogenetically, closely related" means representatively plants categorized into the same order, preferably categorized into the same family, more preferably categorized into the same genus, and even more preferably categorized into the same species. Tapetum is present in the innermost layer of an anther and has essential functions in the development of pollen, which are shared by substantially all spermatophytes. Considering this point, it could be easily understood that transcription factors having the same or similar functions to that of ZPT3-2 may be widely present in other plants.

As a technique for suppressing the expression of an endogenous gene, cosuppression and antisense techniques may be utilized, representatively. As to cosuppression, when a recombinant gene is introduced into a plant cell, the expression of both the gene itself and an endogenous gene including a sequence homologous to part of that gene are suppressed. When cosuppression is utilized, an expression cassette according to the present invention includes DNA encoding a transcription factor or a fragment thereof in the form linked in a forward direction with respect to the promoter. After DNA encoding a transcription factor or a fragment thereof is introduced into a plant cell as an expression cassette, the DNA or fragment thereof can be transcribed in the sense direction under control of the promoter. Due to the action of the introduced DNA, it is possible to suppress the targeted gene expression. Cosuppression can be observed in some transformed plant individuals, but mostly, cosuppression does not occur sufficiently in other individuals. Therefore, typically, individuals in which gene expression is suppressed in an intended manner are screened with routine procedures.

Antisense means that when a recombinant gene is introduced into a plant cell, the transcribed product (mRNA) of the introduced gene forms a hybrid with the complementary sequence of the transcribed product (mRNA) of an endogenous gene so that the translation of a protein encoded by the endogenous gene is inhibited. When antisense is utilized, the expression cassette of the present invention includes DNA encoding a transcription factor or a fragment thereof in the form linked in a reverse direction with respect to the promoter. After DNA encoding a transcription factor or a fragment thereof is introduced into a plant cell as an expression cassette, the DNA or fragment thereof may be transcribed in the antisense direction under control of the promoter. Due to the action of the antisense transcripts, it is possible to suppress the expression of the targeted gene.

### (Promoter derived from ZPT3-2 gene)

A promoter useful in a method for producing a plant having a modified trait according to the second aspect of the invention is a promoter which includes any of (a) DNA having a sequence from position 1 to position 1991 of a base sequence indicated by SEQ ID NO: 3; and (b) DNA having a part of the sequence (a) and which exhibits promoter activity specific to the tapetum of an anther. The above-described promoter of the present invention is preferably the promoter of (a), i.e., the promoter for the ZPT3-2 gene.

A sequence having promoter activity specific to a tapetum, which is obtained by removing a sequence which is not essential for tissue-specific expression activity from the promoter regions for the ZPT3-2 gene, falls within the scope of the present invention. Such a sequence can be obtained by conducting a promoter deletion experiment in accordance with a commonly used method. Briefly, a plasmid obtained by fusing various promoter region deletion mutants of the ZPT3-2 gene (e.g., mutants obtained by deleting the promoter region from the 5' upstream side of the ZPT3-2 gene in various lengths), and an appropriate reporter gene (e.g., the GUS gene) can be used to measure the tissue-specific promoter activity of the deletion mutants, thereby identifying a region essential for the activity.

Once the region essential for the promoter activity is identified, it is possible that a sequence within or adjacent to the region is modified so that the magnitude of the expression activity of the promoter is increased. The thus-obtained variants also fall within the present invention as long as the variants exhibit promoter activity specific to a tapetum.

In the present invention, "exhibit promoter activity specific to a tapetum" means that the ability of a promoter to initiate the transcription of DNA to direct gene expression in a naturally-occurring plant or a plant to which the promoter is introduced as an expression cassette in which the promoter is linked to an arbitrary structural gene, is exhibited specifically in a tapetum. Here, "specific" means that the expression activity of a promoter is higher than in all the other tissues of the flower of the same plant (including pollen, filament, style, petal, calyx, and the like). The above-described specific promoter preferably has an expression activity in a tapetum, higher than the expression activity in all the other tissues of the flower and portions other than the flower (roots, leaves, stems, and the like) of the same plant. More preferably, the specific promoter exhibits substantially no activity in all the other tissues of the flower and portions other than the flower of the same plant. The magnitude of expression activity may be evaluated by comparing the expression level of a promoter in a tapetum with the expression level of the same promoter in other flower tissues in accordance with a commonly used method. The expression level of a promoter is typically determined by the production amount of the products of a gene expressed under control of the promoter.

The conferring of male sterility to a plant using the above-described specific promoter can be achieved as a result of the tapetum-specific expression of any heterologous gene operatively linked to the promoter of the present invention under the control of the promoter in a transformed plant into which the gene has been introduced. It is well known that in a number of tissue-specific promoters, the tissue-specificity is conserved among species. Therefore, it is easily understood that the promoter of the present invention can be applied to a wide variety of plant species.

For example, the promoter of the present invention can be obtained by screening the genomic library of a plant using known cDNA as a probe, and isolating an upstream sequence of a coding region from the corresponding genomic clone. As an example of cDNA, cDNA of the above-described ZPT3-2, which is a transcription factor derived from Petunia, is illustrated.

The promoter of the present invention is not limited to that isolated from the nature, but may include synthesized polynucleotides. For example, synthesized polynucleotides may be obtained by synthesizing or modifying the sequence of a promoter sequenced as described above or an active region thereof with a method well-known to those skilled in the art.

### (Construction of expression cassette and expression vector)

DNA encoding the transcription factor of the present invention can be introduced into plant cells as an expression cassette, in which the DNA is operatively linked to an appropriate promoter using a method well known to those skilled in the art, with a known gene recombinant technique. Similarly, the tapetum-specific promoter of the present invention can be introduced into plant cells as an expression cassette in which the promoter is operatively linked to a desired heterologous gene.

A "promoter" which can be linked to the above-described transcription factor means any promoter which expresses in plants, including any of a constitutive promoter, a tissue-specific promoter, and an inducible promoter.

"Constitutive promoter" refers to a promoter which causes a structural gene to be expressed at a certain level irrespective of stimuli inside or outside plant cells. When a heterologous gene is expressed in other tissues or organs of a plant and a plant is not given an undesired trait, use of a constitutive promoter is simple and preferable. As examples of such a constitutive promoter, 35S promoter (P35S) of cauliflower mosaic virus (CaMV), and the promoter for nopaline synthase (Tnos) are illustrated, but the constitutive promoter is not limited to these.

In the present invention, "tissue-specific promoter" refers to a promoter which causes a structural gene to be expressed specifically in at least a tissue of an anther including a tapetum. Such a tissue-specific promoter includes the promoter derived from the ZPT3-2 gene of the present invention and, in addition, other known promoters having anther-specific expression activity. Therefore, use of an expression cassette of the naturally-occurring ZPT3-2 gene comprising a tapetum-specific promoter and a sequence encoding a transcription factor optionally combined with another regulatory element, falls within the present invention.

"Inducible promoter" refers to a promoter which causes a structural gene to be expressed in the presence of a particular stimulus, such as chemical agents, physical stress, and the like, and which does not exhibit expression activity in the absence of the stimulus. As an example of such an inducible promoter. a glutathione S-transferase (GST) promoter which can be induced by auxin (van der Kop, D. A. et al., Plant Mol. Biol., 39:979, 1999) is illustrated, but the inducible promoter is not limited to this.

In the present specification, the term "expression cassette" or "plant expression cassette" refers to a nucleic acid sequence including DNA encoding the transcription factor of the present invention and a plant expression promoter operatively (i.e., in such a manner that can control the expression of the DNA) linked to the DNA, and a nucleic acid sequence including the tapetum-specific promoter of the present invention and a heterologous gene operatively (i.e., in-frame) linked to the promoter.

"Heterologous gene" which may be linked to the above-described tapetum-specific promoter refers to any of endogenous genes of Petunia other than the ZPT3-2 gene, endogenous genes in a plant other than Petunia, or genes exogenous to plants (e.g., genes derived from animals, insects, bacteria, and fungi), where the expression of products of such a gene are desired in a tapetum. A preferable example of such a heterologous gene in the present invention is a gene which encodes a cytotoxic gene product and whose expression inhibits the development of pollen. As a specific example of such a gene, the barnase gene (Beals, T. P. and Goldberg, R. B., Plant Cell, 9:1527, 1997) is illustrated, but the present invention is not limited to this.

"Plant expression vector" refers to a nucleic acid sequence including an expression cassette and, in addition, various regulatory elements linked to the cassette in such a manner that the regulatory elements can be operated in host plant cells. Preferably, such a plant expression vector may include a terminator, a drug-resistant gene, and an enhancer. It is well known matter to those skilled in the art that the types of plant expression vectors and the types of regulatory elements used may be varied depending on host cells. Plant expression vectors used in the present invention may further have a T-DNA region. The T-DNA region increases the efficiency of gene introduction, particularly when Agrobacterium is used to transform a plant.

"Terminator" is a sequence which is located downstream of a region encoding a protein of a gene and which is involved in the termination of transcription when DNA is transcribed into mRNA, and the addition of a poly Asequence. It is known that a terminator contributes to the stability of mRNA, and has an influence on the amount of gene expression. As examples of such a terminator, the terminator for the nopaline synthase gene (Tnos), and the 35S terminator of cauliflower mosaic virus (CaMV) are illustrated, but the terminator is not limited to these.

"Drug-resistant gene" is desirably one that facilitates the selection of transformed plants. The neomycin phosphotransferase II (NPTII) gene for conferring kanamycin resistance, and the hygromycin phosphotransferase gene for conferring hygromycin resistance may be preferably used, but the drug-resistant gene is not limited to these.

The plant expression vector of the present invention may be prepared using a gene recombinant technique well known to those skilled in the art. A plant expression vector is constructed, for example, preferably using pBI-type vectors or pUC-type vectors, but the plant expression vector is not limited to these.

### (Production of transformed plant)

The thus-constructed expression cassette, or an expression vector including the expression cassette, may be introduced into desired plant cells using a known gene recombinant technique. The introduced expression cassette is present to be integrated into DNA in a plant cell. It should be noted that DNA in a plant cell includes not only chromosome but also DNA included in various organelles included in a plant cell (e.g., a mitochondria, and a chloroplast).

In the present specification, the term "plant" includes any of monocotyledons and dicotyledons. Preferable plants are dicotyledons. Dicotyledons include any of Archichlamiidae and Sympetalidae. A preferable subclass is Sympetalidae. Sympetalidae includes any of Gentianales, Solanales, Lamiales, Callitrichales, Plantaginales, Campanulales, Scrophulariales, Rubiales, Dipsacales, and Asterales. A preferable order is Solanales. Solanales includes any of Solanaceae, Hydrophyllaceae, Polemoniaceae, Cuscutaceae, and Convolvulaceae. A preferable family is Solanaceae. Solanaceae includes Petunia, Datura, Nicotiana, Solanum, Lycopersicon, Capsicum, Physalis, Lycium, and the like. Preferable genera are Petunia, Datura, and Nicotiana, and more preferably Petunia. The genus Petunia includes the following species: P. hybrida, P. axillaris, P. inflata, P. violacea, and the like. A preferable species is P. hybrida. "Plant" means phanerogamic plants and seed obtained from the plants unless otherwise specified.

As examples of "plant cells", cells in each tissue of plant organs, such as flowers, leaves, roots, and the like, callus, and suspension cultured cells are illustrated.

For the purpose of introduction of a plant expression vector into a plant cell, a method well known to those skilled in the art, such as an indirect method using Agrobacterium, and a method for directly introducing into cells, can be used. As such an indirect method using Agrobacterium, for example, a method of Nagel et al. (FEMS Microbiol. Lett., 67:325 (1990)) may be used. In this method, initially, Agrobacterium is transformed with a plant expression vector (e.g., by electroporation), and then the transformed Agrobacterium is introduced into a plant cell with a well-known method, such as a leaf disk method and the like. As a method for directly introducing a plant expression vector into a cell, an electroporation method, particle gun, a calcium phosphate method, a polyethylene glycol method, and the like are illustrated. These methods are well known in the art. A method suitable for a plant to be transformed can be appropriately selected by those skilled in the art.

Cells into which a plant expression vector has been introduced are screened for drug resistance, such as kanamycin resistance and the like, for example. A selected cell may be regenerated to a plant using a commonly used method.

Whether or not an introduced plant expression vector is operative in a regenerated plant can be confirmed with a technique well-known to those skilled in the art. For example, in the case where suppression of the expression of an endogenous gene is intended, such confirmation can be conducted by measuring the level of transcription with Northern blot analysis. In this manner, a desired transformed plant in which the expression of an endogenous transcription factor is suppressed can be selected. For the purpose of the expression of a heterologous gene using a tissue-specific promoter, the expression of the heterologous gene can be confirmed usually by Northern blot analysis using RNA extracted from a target tissue as a sample. The procedures of this analysis method are well known to those skilled in the art.

Whether or not the expression of an endogenous transcription factor is suppressed in accordance with the method of the present invention so that pollen fertility is reduced can be confirmed, for example, by observing the form of the pollen of a plant, which is transformed by an expression vector including DNA encoding a transcription factor, with a microscope optionally after histochemically staining.

Whether or not a promoter is expressed specifically in a tapetum in accordance with the method of the present invention can be confirmed by, for example, histochemically staining flower tissues including the anther in a plant transformed with an expression vector, in which a promoter is operatively linked to the GUS gene, by a commonly used method to detect the distribution of GUS activity.

### (Examples)

Hereinafter, the present invention will be described based on examples. The scope of the present invention is not limited to the examples only. Restriction enzymes, plasmids, and the like used in the examples are available from commercial sources.

### (Example 1: Construction of plant expression vector including polynucleotide encoding ZPT3-2)

Out of the previously reported anther-specific ZF genes (Kobayashi et al., above), cDNAs of PEThy ZPT3-2 (ZPT3-2) was linked downstream of the 35S promoter of the cauliflower mosaic virus to prepare a plant expression vector.

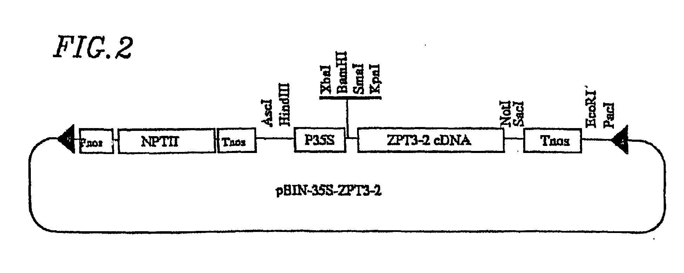

Specifically, DNA fragments including the cauliflower mosaic virus 35S promoter (HindIII-XbaI fragment) and DNA fragments including the NOS terminator (SacI-EcoRI fragment) in plasmid pBI221 (purchased from CLONTECH Laboratories Inc.) were successively inserted into the multi-cloning site of plasmid pUCAP (van Engelen, F. A. et al., Transgenic Res., 4:288, 1995) to prepare pUCAP35S. A pBluescript vector including cDNA of ZPT3-2 was cleaved at KpnI and SacI sites (either is a site within the vector), and inserted between KpnI and SacI sites of the above-described pUCAP35S. Further, this recombinant plasmid was cleaved with EcoRI and HindIII, and a DNA fragment encoding ZPT3-2 was inserted between EcoRI and HindIII sites of binary vector pBINPLUS (van Engelen, F. A. et al., above). As shown in Figure **2**, the constructed ZPT3-2 gene comprises the 35S promoter region (P35S; 0.9 kb) of cauliflower mosaic virus (CaMV), a polynucleotide (ZPT3-2; about 1.9 kb) encoding ZPT3-2 of the present invention, and the terminator region of nopaline synthase (Tnos; 0.3 kb). Pnos in Figure **2** indicates the promoter region of nopaline synthase, and NPTII indicates the neomycin phosphotransferase II gene.

### (Example 2: Isolation of ZPT3-2 promoter region and linkage to GUS reporter gene)

cDNA of ZPT3-2 was used as a probe to isolate corresponding genomic clones from the genome DNA library of Petunia. A DNA fragment (promoter region; about 2.0 kb) upstream of the transcription initiation site was subcloned. This DNA fragment was linked upstream of the GUS reporter gene and cloned into a binary vector.

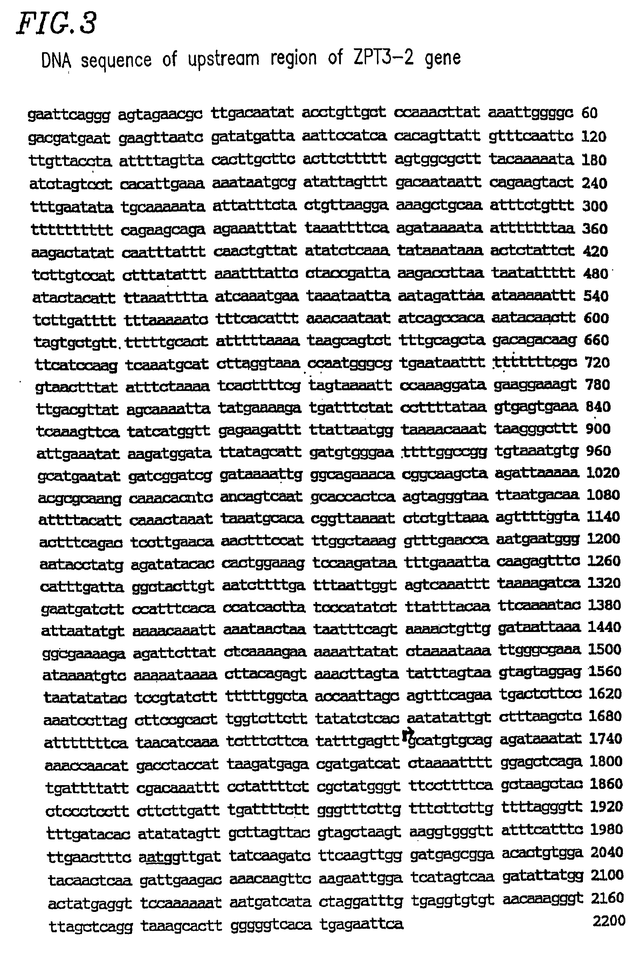

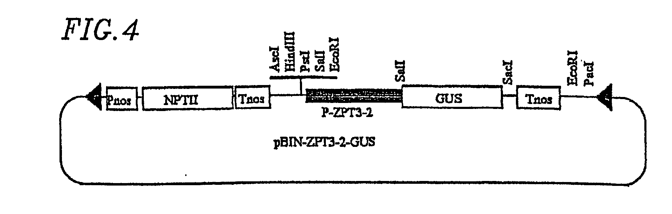

Specifically, cDNA of ZPT3-2 was labeled with [α-³²P]dCTP using a commonly used random priming method (Sambrook et al., above) to prepare a radiolabeled probe. With this probe, a genomic library of Petunia (Petunia hybrida var. Mitchell) prepared within EMBL3 vector (manufactured by Stratagene) was screened. A genome DNA fragment of about 2.0 kb including the upstream region of the gene from the resultant clone was subcloned at the EcoRI site of pBluescriptSK vector (pBS-ZPT3-2-E), followed by sequencing (Figure **3**). Next, a linker DNA including a SalI recognition sequence was inserted in BglII site (base position 2006) in the upstream sequence of the ZPT3-2 gene, so that SalI site was introduced therein (base position: 2016). Thereafter, a DNA fragment cleaved with EcoRI and SalI was inserted upstream of the GUS coding region of pUCAPGUSNT (pUCAP-ZPT3-2-GUSNT). Therefore, the ZPT3-2 gene was connected to the GUS coding region in frame at a region near the N terminus of the coding region of the ZPT3-2 gene. Further, a DNA fragment obtained by cleaving pUCAP-ZPT3-2-GUSNT with EcoRI and HindIII (including the ZPT3-2 promoter, the GUS coding region and the NOS terminator) was inserted into pBINPLUS vector to obtain pBIN-ZPT3-2-GUS (Figure **4**).

### (Example 3: Introduction of each fusion gene into Petunia cells)

Each of the above-described expression vectors was introduced via Agrobacterium into Petunia (Petunia hybrida var. Mitchell) with the following procedures.
(1) Agrobacterium tumefaciens LBA4404 strain (purchased from CLONTECH Laboratories Inc.) was cultured at 28°C in L medium containing 250mg/ml of streptomycin and 50mg/ml of rifampicin. Cell suspension was prepared in accordance with the method of Nagel et al. (above). The plasmid vector constructed in Examples 1 and 2 were introduced into the above-described strain by electroporation.
(2) A polynucleotide encoding each fusion gene was introduced into Petunia cells using the following method: the Agrobacterium tumefaciens LBA4404 strain obtained in the above-described (1) was shake-cultured (28°C, 200 rpm) in YEB medium (DNA Cloning, Vol. 2, page 78, Glover D. M. Ed., IRL Press, 1985). The resultant culture was diluted with sterilized water by a factor of 20, and cocultured with leaf pieces of Petunia (Petunia hybrida var. Mitchell). After 2 to 3 days, the above-described bacterium was removed in medium containing antibiotics. The Petunia cells were subcultured with selection medium every two weeks. The transformed Petunia cells were selected based on the presence or absence of kanamycin resistance due to the expression of the NPTII gene derived from pBINPLUS which had been introduced along with the above-described two fusion genes. The selected cells were induced into callus with a commonly used method. The callus was redifferentiated into a plant (Jorgensen R. A. et al., Plant Mol. Biol., 31:957, 1996).

### (Example 4: Phenotype of transformed Petunia into which ZPT3-2 gene is introduced)

The transformants obtained by introducing the vector of Example 1 were used to measure the expression amount of ZPT3-2 and observe the form of pollen, so that the action and effect of introducing cDNA of ZPT3-2 into a plant were examined.

Specifically, from transformants (15 individuals) into which cDNA of ZPT3-2 had been introduced under the control of 35S promoter, individuals (4 individuals) in which gene expression was suppressed by cosuppression were selected by Northern blot analysis. The conditions of the Northern blot analysis were the following: hybridization was conducted in a solution containing 7% SDS, 50% formamide, 5×SSC, 2% blocking reagent (manufactured by Boehringer Mannheim), 50 mM sodium phosphate buffer (pH 7.0), 0.1% sodium lauryl sarcosine, 50 µg/ml of yeast tRNA, and ³²P-labeled probe DNA (1×10⁷ cpm) at 68°C for 16 hours, followed by washing with 2×SSC/0.1% SDS at 68°C for 30 minutes.

In the above-described four cosuppressed transformants, about 90% of their pollen cells were dead before mature. With a commonly used flavonol staining, flanovol which is normally contained in the exine layer of the surface of a pollen cell was not substantially detected in the destroyed cells of these transformants (Figure **5**; grains having bluish white irregular forms in photographs showing the pollen of the transformants are the disrupted cells).

According to the above-described observed results, it is speculated that in the pollen cells of the transformants in which the gene expression was inhibited, the development of the cell wall was insufficient due to abnormality in the accumulation of the exine layer, so that cells which could not withstand osmotic pressure were disrupted. No difference was recognized between the development of the pollen cells of the transformants and that of a normal plant until the tetrad stage. The time when abnormality occurs in pollen coincides with the time when the exine layer is accumulated and the time when the ZPT3-2 is normally expressed, which supports the above-described action mechanism. In the transformants in which gene expression was inhibited, no change in traits other than pollen was observed. It should be noted that no change in pollen or other traits was recognized in individuals in which the ZPT3-2 gene was excessively expressed.

As described above, the introduction of a gene encoding ZPT3-2 leads to the inhibition of the development of pollen at a high rate, thereby making it possible to eliminate fertility. Such a rate can be further enhanced by optimizing a promoter which is used for gene introduction. For example, instead of the CaMV35S promoter used alone in the vector in Example 1, two CaMV35S promoters may be linked together in tandem to enhance the activity (so called E2 promoter) and the resultant promoter may be utilized. The introduction of the ZPT3-2 gene may be useful as a selective transformation technique, since the effect of the introduction is specific to pollen and the other traits of a plant is not affected.

### (Example 5: Tissue specificity of promoter activity of ZPT3-2)

The tissue-specific promoter activity of the above-described DNA fragment was detected by histochemical staining with GUS activity using the transformants obtained by introducing the vector in Example 2.

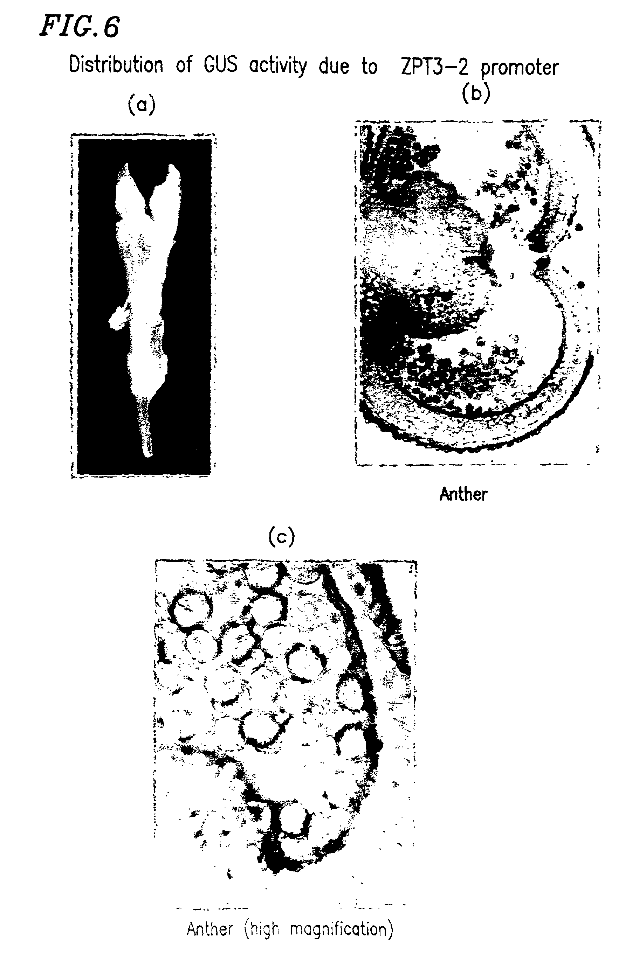

Specifically, the flowers of the transformants obtained by introducing a fusion gene of the upstream region of the ZPT3-2 gene with GUS were used to study the distribution of GUS activity using X-GUS as a substrate (Gallagher, S. R. Ed., GUS protocols: using the GUS gene as a reporter of gene expression, Academic Press, Inc., pp. 103-114, 1992). As a result, GUS activity was detected specifically in the tapetum of an anther (Figure **6**). The time of expression was transient. The peak of activity substantially corresponded to the tetrad stage immediately after the meiosis of a pollen cell. Therefore, it was found that the promoter for the ZPT3-2 gene exhibits activity specific to the tapetum of an anther.

As described above, the promoter for the ZPT3-2 gene exhibits activity specific to the tapetum around the tetrad stage. As described above, a tapetum is a tissue which plays an essential role in the development of pollen. Therefore, this promoter is useful as a tool for detailed study of the development of pollen. Further, the development of pollen can be inhibited by using the promoter or an active fragment thereof to specifically express a gene having cytotoxicity or the like to destroy the tissue or function of a tapetum.

### INDUSTRIAL APPLICABILITY

The method of the present invention utilizing DNA encoding a transcription factor derived from the ZPT3-2 gene, and a promoter derived from the ZPT3-2 gene is useful as a technique for selectively modifying the trait of a plant using a genetic engineering method, particularly a technique for conferring male sterility.

## Claims

1. A method for producing a male sterile plant, comprising the steps of:
providing a plant expression cassette including: a nucleic acid being any of (i) DNA having a sequence from position 1 to position 1886 of a base sequence indicated by SEQ ID NO: 1, (ii) DNA hybridizing the DNA having the base sequence (i) under stringent conditions and encoding a transcription factor controlling the development of pollen, and (iii) a DNA fragment of (i) or (ii); and a promoter operatively linked to the nucleic acid;
providing plant cells having an endogenous transcription factor controlling the development of pollen, wherein a gene encoding the endogenous transcription factor hybridizes the nucleic acid under stringent conditions;
introducing the expression cassette into the plant cells;
regenerating the plant cells, into which the expression cassette has been introduced, to plants; and
screening the regenerated plants for one in which the nucleic acid is expressed so that expression of the endogenous transcription factor is suppressed.

2. A method according to claim 1, wherein the nucleic acid is linked in a forward direction with respect to the promoter, and is transcribed in a sense direction in cells of the plant.

3. A method according to claim 1, wherein the nucleic acid is linked in a reverse direction with respect to the promoter, and is transcribed in a antisense direction in cells of the plant.

4. A method according to claim 1, wherein the plant is dicotyledon.

5. A method according to claim 4, wherein the plant is of the family Solanaceae.

6. A method according to claim 5, wherein the plant is of the genus Petunia.

7. A method according to claim 1, wherein the expression cassette is incorporated into a plant expression vector.

8. A male sterile plant produced by a method according to any of claims 1 through 7.

9. A method for conferring male sterility to a plant, comprising the steps of:
providing a plant expression cassette including: a nucleic acid being any of (i) DNA having a sequence from position 1 to position 1886 of a base sequence indicated by SEQ ID NO: 1, (ii) DNA hybridizing the DNA having the base sequence (i) under stringent conditions and encoding a transcription factor controlling the development of pollen, and (iii) a DNA fragment of (i) or (ii); and a promoter operatively linked to the nucleic acid;
providing plant cells having an endogenous transcription factor controlling the development of pollen, wherein a gene encoding the endogenous transcription factor hybridizes the nucleic acid under stringent conditions;
introducing the expression cassette into the plant cells;
regenerating the plant cells, into which the expression cassette has been introduced, to plants; and
screening the regenerated plants for one in which the nucleic acid is expressed so that expression of the endogenous transcription factor is suppressed.

10. A method for producing a male sterile plant, comprising the steps of:
providing a plant expression cassette including: a promoter including any of (a) DNA having a sequence from position 1 to position 1991 of a base sequence indicated by SEQ ID NO: 3 and (b) DNA having a part of the sequence of (a) and exhibiting promoter activity specific to the tapetum of an anther; and a heterologous gene operatively linked to the promoter;
introducing the expression cassette into plant cells; and
regenerating the plant cells, into which the expression cassette has been introduced, to plants.

11. A method according to claim 10, wherein the plant is dicotyledon.

12. A method according to claim 11, wherein the plant is of the family Solanaceae.

13. A method according to claims 12, wherein the plant is of the genus Petunia.

14. A method according to claim 10, wherein the expression cassette is incorporated into a plant expression vector.

15. A male sterile plant produced by a method according to any of claims 10 through 14.

16. A method for conferring male sterility to a plant, comprising the steps of:
providing a plant expression cassette including: a promoter including any of (a) DNA having a sequence from position 1 to position 1991 of a base sequence indicated by SEQ ID NO: 3 and (b) DNA having a part of the sequence of (a) and exhibiting promoter activity specific to the tapetum of an anther; and a heterologous gene operatively linked to the promoter;
introducing the expression cassette into plant cells; and
regenerating the plant cells, into which the expression cassette has been introduced, to plants.

17. A promoter comprising DNA of the following (I) or (II):
(I) DNA having a sequence from position 1 to position 1991 of a base sequence indicated by SEQ ID NO: 3 and (II) DNA having apart of the sequence of (I) and exhibiting promoter activity specific to a tapetum of an anther.

18. A plant expression cassette useful for conferring male sterility to a plant, comprising a promoter according to claim 17 and a heterologous gene operatively linked to the promoter.
